# EUROPEAN PATENT SPECIFICATION

(11) **EP 1 633 433 B1**
(45) Date of publication and mention of the grant of the patent: **28.04.2010**
(21) Application number: 04755007.4
(22) Date of filing: 10.06.2004
(51) Int. Cl.: A61N 1/36, A61F 9/00

(54) **IMPLANT INSTRUMENT**
EINSETZINSTRUMENT
INSTRUMENT POUR IMPLANT

(30) Priority: 13.06.2003 US 462224
(43) Date of publication of application: 15.03.2006
(73) Proprietor: IMI Intelligent Medical Implants AG, 6304 Zug (CH)
(72) Inventor: CHOW, Alan, Y., Wheaton, IL 60187 (US); CHOW, Vincent, Hanover Park, IL 60103 (US); FLORY, Eron, Austin, TX 78703 (US); GINSBURG, Leonard, M., Berkley, CA 94705 (US); WANG, Carl, C., T., Oakland, CA 94619 (US)
(74) Representative: Graf von Stosch, Andreas
(86) International application number: PCT/US2004/018604
(87) International publication number: WO 2004/112893

(56) References cited:
- US-A- 5 730 718
- US-A- 5 871 492
- US-B1- 6 389 317

## Description

### FIELD OF THE INVENTION

The present invention relates to instruments for use with medical implants and, in particular, to instruments for inserting implants into the eye.

### BACKGROUND

A variety of retinal diseases cause vision loss by destruction of the outer retinal vasculature and certain outer and inner retinal layers of the eye. The inner retina is also known as the neuroretina. The outer retinal vasculature is comprised of the choroid and choriocapillaris, and the outer retinal layers are comprised of Bruch's membrane and retinal pigment epithelium. The outer portion of the inner retinal layer that is affected is the photoreceptor layer. Variable sparing of other inner retinal layers, however, may occur. These spared inner retinal layers include the layers of the outer nuclei, outer plexiform, inner nuclei, inner plexiform, amacrine cells, ganglion cells, and the nerve fibers. The sparing of these inner retinal layers allows electrical stimulation of one or more of these structures to produce sensations of formed images.

Prior efforts to produce vision by electrically stimulating various portions of the retina have been reported. One such attempt involved a disk-like device with retinal stimulating electrodes on one side and photosensors on the other side. The photosensor current was to be amplified by electronics (powered by an external source) within the disk to power the stimulating electrodes. The device was designed to electrically stimulate the retina's nerve fiber layer via contact upon this layer from the vitreous cavity.

Another early attempt at using an implant to correct vision loss involves a device consisting of a supporting base onto which a photosensitive material, such as selenium, is coated. This device was designed to be inserted through an external sclera incision made at the posterior pole and would rest between the sclera and choroid, or between the choroid and retina. Light would cause an electric potential to develop on the photosensitive surface producing ions that would then theoretically migrate into the retina causing stimulation.

More recently, so-called sub-retinal implants have been proposed. In particular, Chow et al. have described various designs for implants to be inserted in the sub-retinal space, i.e. a space created between the inner and outer retinal layers, in U.S. Patent Nos. 5,016,633; 5,024,223; 5,397,350; 5,556,423; 5,895,415; 6,230,057; 6,389,317 and 6,427,087. Generally, the implants described in these patents are placed in contact with the photoreceptor layer of the inner retina such that electrodes on the implants can provide stimulating currents, derived from the photovoltaic conversion of incident light, to the inner retina. Additionally, techniques and devices for inserting such implants into the sub-retinal space are also described in various ones of these patents, e.g. U.S. Pat. Nos. 5,016,633; 5,024,223 and 6,389,317. While some of these techniques and, more particularly, devices, have been effectively used to implant sub-retinal devices in the past, a need exists for improved techniques and devices to further simplify delivery of implants, particularly sub-retinal implants.

US. Patent 5, 871, 492 describes on other hand a device to handle a mammalian eye having also a housing, a longitudinal channel as mentionned in claim 1 but does not disclose an instrument for use with an implant.

### BRIEF SUMMARY OF THE INVENTION

According to one aspect, the present invention provides an instrument as defined in claim 1 for use with a medical implant, and particularly for use in implanting a retinal implant. Preferred features of the invention are recited in the dependent claims. Thus, the present invention relates to an instrument for use with medical and other implants, particularly for use in implanting retinal implants into the sub-retinal space of an eye. In one embodiment, the instrument includes a handpiece having a first sliding member disposed within a longitudinal channel defined by a housing. A nose member comprising a biasing surface is coupled to the housing. The first sliding member comprises legs longitudinally extending through the channel and the nose member, each leg further comprising an engaging surface. Movement of the first sliding member (which is preferably biased to an initial, retracted position) along the channel in the direction of the nose member causes the engaging surface of each leg to contact the biasing surface of the nose member such that each leg is biased inwardly. Finger portions disposed at the distal end of each leg are thereby controlled to engage surfaces of an inserter attachment to maintain the inserter attachment in a fixed relationship relative to the nose member. A button member is provided to allow user-actuated movement of the first sliding member. Additionally, a lever and linkage arrangement is preferably provided to impart longitudinal or axial movement of a second sliding member disposed within the housing. In one embodiment, a slidable trigger lock is provided that, when engaged, prevents movement of the lever and linkage. In another embodiment, a surface of the nose member comprises at least two recesses defined therein such that one of the two recesses engages a projecting member of the inserter attachment in order to maintain the inserter attachment at a selected alignment relative to the nose member. Preferably, the at least two recesses are arranged at different angular positions so as to accommodate user preferences.

In another embodiment, an inserter attachment comprises a body member having a longitudinal channel defined therein, and a conduit coupled to the longitudinal channel. A conduit linkage is provided within the conduit, the conduit linkage terminating in a pusher cap at an end proximate to the body member and terminating in a pusher at the distal end of the conduit. A resilient member is disposed between the body member and the pusher cap in order to bias the pusher into an initial, retracted position. In one embodiment, the pusher cap, when the inserter attachment is coupled to the handpiece, engages the second sliding member such that longitudinal movement of the second sliding member within the housing is translated, via the conduit linkage of the inserter attachment, to movement of the pusher at the distal end of the conduit. When an implant is positioned at the distal end of the conduit and in contact with the pusher, such movement may be used to move the implant out of the inserter attachment and into, for example, the sub-retinal space of an eye.

In yet another embodiment, an open-ended tray is coupled to the distal end of the conduit such that the pusher rests in the open-ended tray. A cover, preferably fashioned from a transparent, compliant material, encompasses the open-ended tray, the pusher and at least some of the conduit. The cover preferably comprises at least two flaps at an open end of the cover, wherein at least one of the at least two flaps substantially overlays the other flaps such that a space defined by the open-ended tray and the cover is at least partially closed by the at least two flaps. Each flap may be tapered in equal or differing amounts.

Preferably, an implant, such as a retinal implant, is disposed within the space so defined and retained within the space by the at least two flaps.

Using the instrument of the present invention, delivery of implants, including sub-retinal (or, more generally, intraocular) implants, is greatly facilitated.

### BRIEF DESCRIPTION OF THE DRAWINGS

FIG. 1 is a simplified cross-sectional side view of an eye containing a retinal implant in the subretinal space;

FIG. 2 is an enlarged exploded perspective sectional view of a portion of the retina illustrating a perspective sectional view of a retinal implant in a preferred location in the subretinal space;

FIG. 3 is a perspective view of a retinal implant injector (RII) for use in implanting a retinal implant;

FIG. 4 is a perspective view of a syringe retinal implant injector (SRI) assembly comprising the RII of FIG. 3 with a retinal implant inside, an attached cannula, and an attached operator controlled fluid filled syringe;

FIG. 5 is a perspective view of an alternative embodiment of the SRI of FIG. 4;

FIG. 6 is a perspective view of an embodiment of an instrument according to the invention and comprising a handpiece and an inserter attachment for use in inserting implants, particularly retinal implants;

FIG. 7 is an exploded perspective view of the handpiece;

FIG. 8 is a cross-sectional side view of the handpiece;

FIG. 9 is a cross-sectional side view of the inserter attachment;

FIG. 10 is a partial cross-sectional side view of an alternative embodiment of the inserter attachment;

FIG. 11 is a magnified perspective view of a nose member of the handpiece;

FIG. 12 is a top view of an alternative embodiment of a stop pin and groove arrangement provided by a slidable trigger lock;

FIG. 13 is a magnified cross-sectional top view of the handpiece, particularly the nose member;

FIG. 14 is a magnified cross-sectional top view of the inserter attachment and handpiece;

FIG. 15 is a magnified partial cross-sectional top view of an alternative embodiment of the nose member;

FIG. 16 is a magnified perspective view of an inserter tip of the inserter attachment; and

FIG. 17 is a magnified cross-sectional side view of the inserter tip.

### DETAILED DESCRIPTION OF THE PRESENTLY PREFERRED EMBODIMENTS

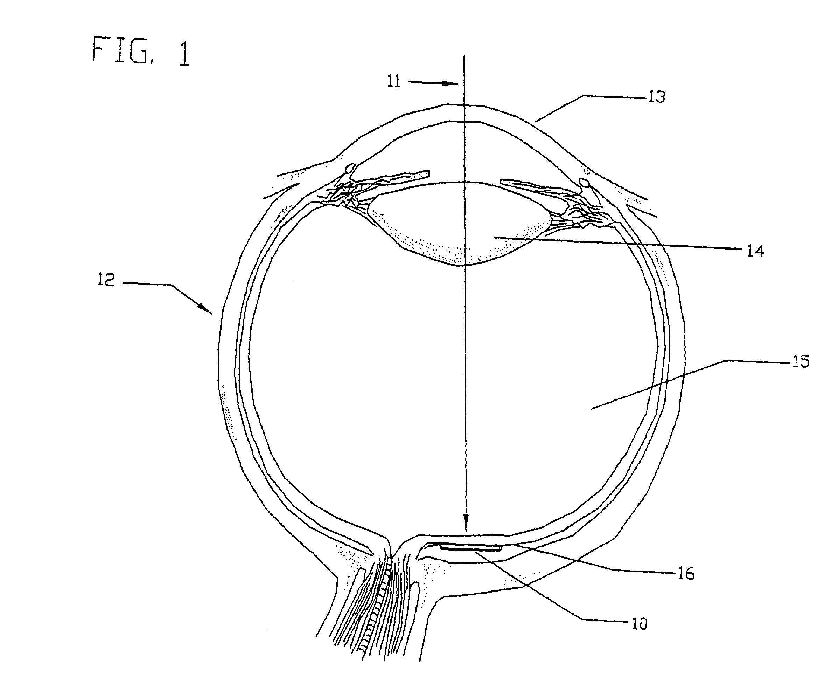

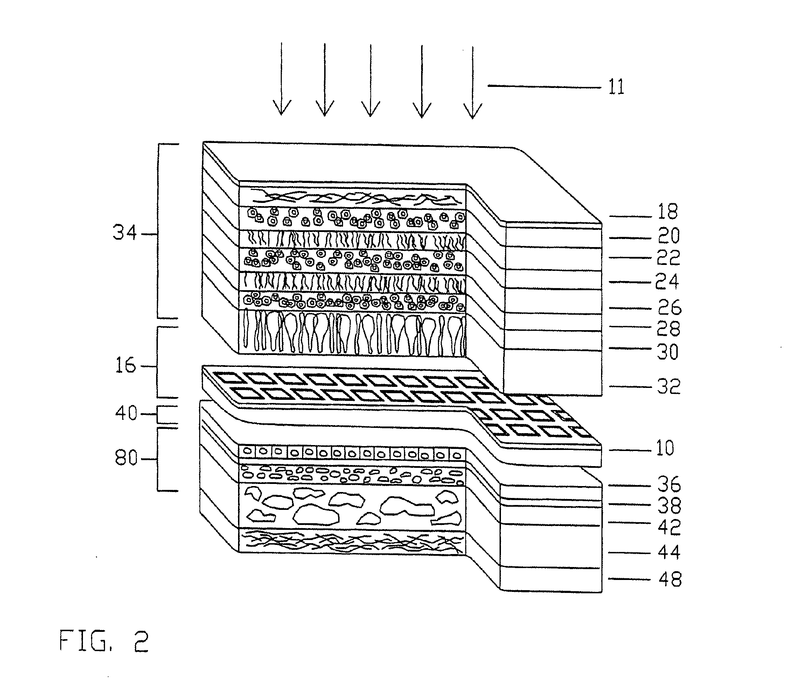

As illustrated in FIG. 1, a retinal implant 10 is positioned inside the eye 12, in the subretinal space 16, and is oriented to receive incident light 11 arriving through the cornea 13 and lens 14 of the eye 12. Note that the positioning of the retinal implant 10 illustrated in FIG. 1 is illustrative only; in practice, the retinal implant 10 may be positioned at various points throughout the sub-retinal space 16 and, in a preferred embodiment, is placed off-axis relative to the macula. As used in this specification, the term light refers to visible and/or infrared light. Preferably, the retinal implant 10 is a photovoltaic device, such as an array of microphotodiodes, for converting the incident light 11 into currents for stimulating the inner retina 34 (FIG. 2). Various embodiments of such devices are taught in U.S. Pat. Nos. 5,016,633; 5,024,223; 5,397,350; 5,556,423; 5,895,415; 6,230,057; 6,389,317 and 6,427,087. In practice, however, the present invention may be more broadly applied to other types of retinal implants or for accessing structures other than the sub-retinal space within the eye. For example, the present invention may be used in conjunction with so-called epiretinal implants, i.e., implants that reside on the inner surface of the inner retina adjacent the vitreous cavity 15. Additionally, the present invention is not limited to photovoltaic or electrical intraocular implants, but may be advantageously used with other types such as, but not limited to, tissue transplants or implants used for drug delivery. Indeed, the present invention may be advantageously applied to other types of medical implants, such as subcutaneous implants. Further still, the present invention need not be limited to use with intraocular or medical implants; the principles described herein may be equally applied to any situation in which an object is to be inserted or otherwise deposited within another material.

In FIG. 2, a high magnification perspective sectional view shows the retinal implant 10 placed in its preferred position in the subretinal space 16. The layers of the retina from inside the eye to the outside in their respective positions are: internal limiting membrane 18; nerve fiber layer 20; ganglion and amacrine cell layer 22; inner plexiform 24; inner nuclear layer 26; outer plexiform 28; outer nuclear layer 30; and photoreceptor layer rod and cone inner and outer segments 32, all of which constitute the inner retina 34. It should be noted that the layers of the outer plexiform 28; outer nuclear layer 30; and photoreceptor layer rod and cone inner and outer segments 32 constitute the outer portion of the inner retina, but are sometimes referred to as just the "outer retina" in the art, although the meaning is clear to one skilled in the art as described in the above context. The implant 10 is disposed between the inner retina 34 and the outer retina 40 comprised of the retinal pigment epithelium 36 and Bruch's membrane 38. External to the outer retina 40 are the choriocapillaris 42 and choroid 44 which together comprise the choroidal vasculature 80. External to the choroidal vasculature 80 is the sclera 48.

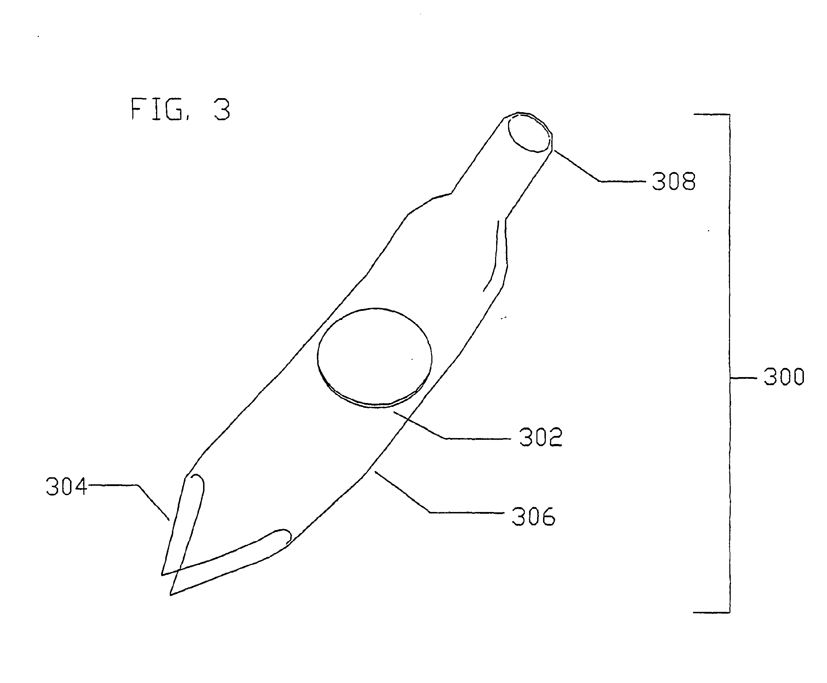

As shown in FIG. 3, a retinal implant injector (RII) 300 may be used to place a retinal implant 302 into the vitreous cavity of the eye, or to place a retinal implant 302 directly into the subretinal space of the eye. The RII 300 employs a fluid, which is placed inside the RII 300, to push the retinal implant 302 to its exit at the terminal tip 304 of the RII 300. By this means, controlled deposition of the retinal implant 302 is possible without physically having to hold the retinal implant 302 with an instrument that can cause damage to the implant 302.

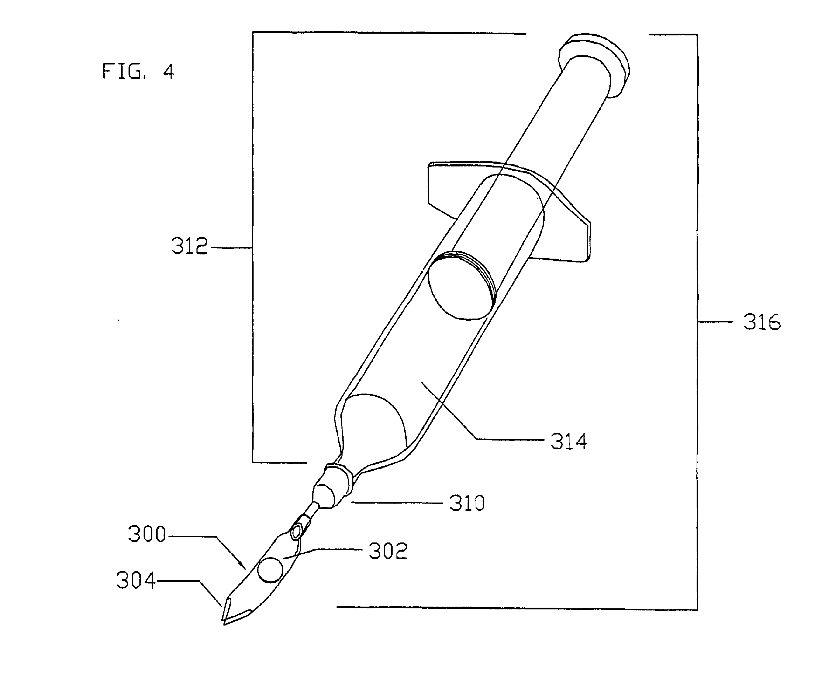

Also shown in FIG. 3, the RII 300 is fabricated from tubing which is preferably made of Teflon (polytetrafluoroethylene) or Parylene and is transparent. It is flattened through most of its length with a taper 304 at the tip of its flattened end. The flattened cross-section 306 preferably is similar to the cross-section of the retinal implant 302. The opposite end of the tube maintains a round cross-section 308 that allows the RII 300 to be inserted around a cannula 310 as shown in FIG. 4 that in turn is attached to a syringe 312 containing the fluid 314 used for the injection. The injection fluid 314 is any biocompatible fluid but is preferably saline or a viscoelastic material.

As shown in FIG. 4, in use, the retinal implant 302 is first placed within the RII 300. The RII 300 is then attached around a cannula 310 that in turn is attached to a syringe 312 containing the preferred saline or viscoelastic fluid. The entire Retinal Injector Assembly 316 is held by the operator via the syringe 312. The tapered tip 304 of the RII 300 is then advanced into the vitreous cavity of the eye through an opening made through the eye wall for this purpose. Once the tip 304 of the RII 300 is placed into position within the vitreous cavity and next to the retinotomy incision made through the retina, the retinal implant 302 is pushed out of the RII 300 by fluid pressure exerted by operation of the fluid filled syringe 312 from outside the eye. The retinal implant is then manipulated with surgical instruments either to a position underneath the retina in the subretinal space, or on top of the retina in the epiretinal position. The RII 300 is also useable to directly inject the retinal implant 302 through the retinotomy opening into the subretinal space. In this case, the tip 304 of the RII 300 is placed directly into the retinotomy opening before injection of the retinal implant 302.

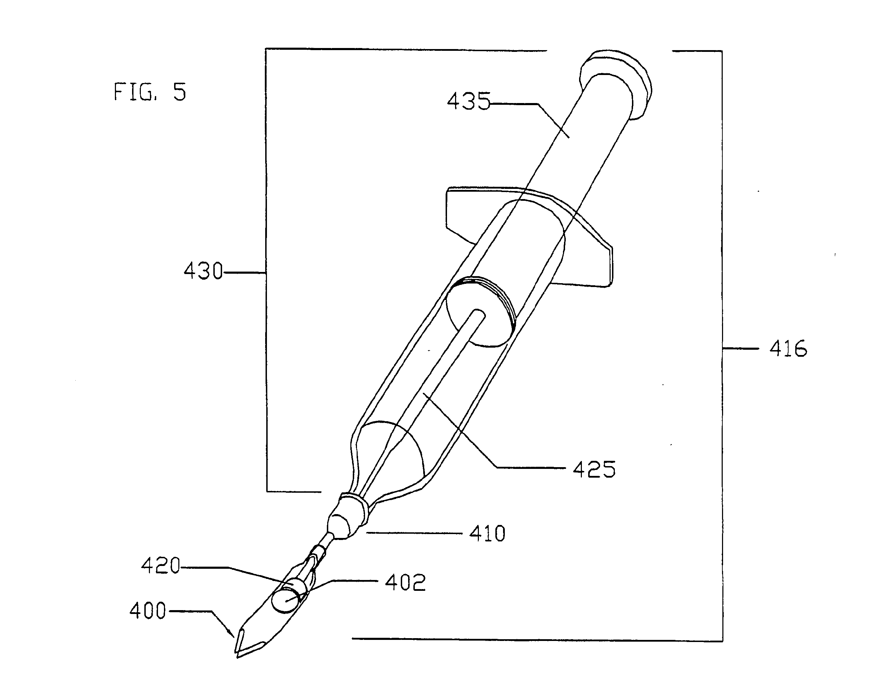

In another embodiment, as shown in FIG. 5, a RII injector assembly 416 utilizes an injector plunger 420, placed within the injector 400, to push the implant 402 out of the injector 400. The injector plunger 420 is shaped to conform to the inside cross-section of the injector 400 and is attached using any variety of well-known methods of moving the plunger 420 forward. In the preferred embodiment, a rod-like extension 425 connects the injector plunger 420 to the syringe plunger 435 of a syringe 430. Pushing the syringe plunger 435 thus pushes the injector plunger 420 forward and moves the implant 402 out of the injector 400.

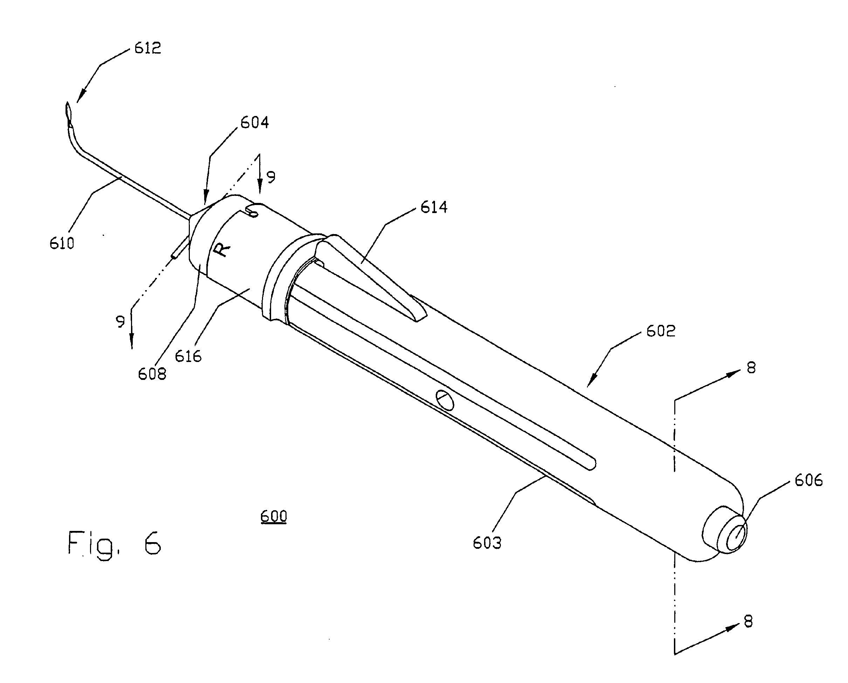

Referring now to FIG. 6, an embodiment of an instrument 600, according to the invention, for use in inserting a retinal implant is illustrated. As shown, the instrument 600 comprises a handpiece 602 and an inserter attachment 604. The handpiece 602 comprises a housing 603. A button member 606 is provided to engage or disengage the inserter attachment 604, as described in greater detail below. The inserter attachment 604 comprises a body member 608 coupled to a conduit 610 that terminates in an inserter tip 612. In a presently preferred embodiment, a retinal implant is positioned within the inserter tip 612, although it is possible that the present invention may be employed for use with other types of implants. As described in further detail below, a lever or trigger 614 is provided to express the implant from the inserter tip 612. To prevent inadvertent movement of the lever 614, a slidable trigger lock 616 is provided.

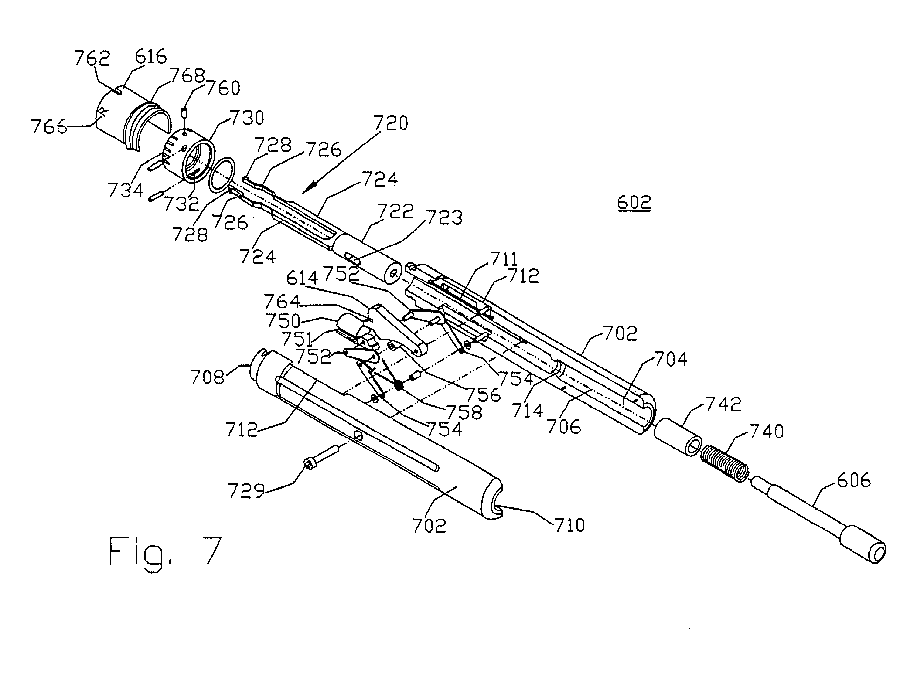

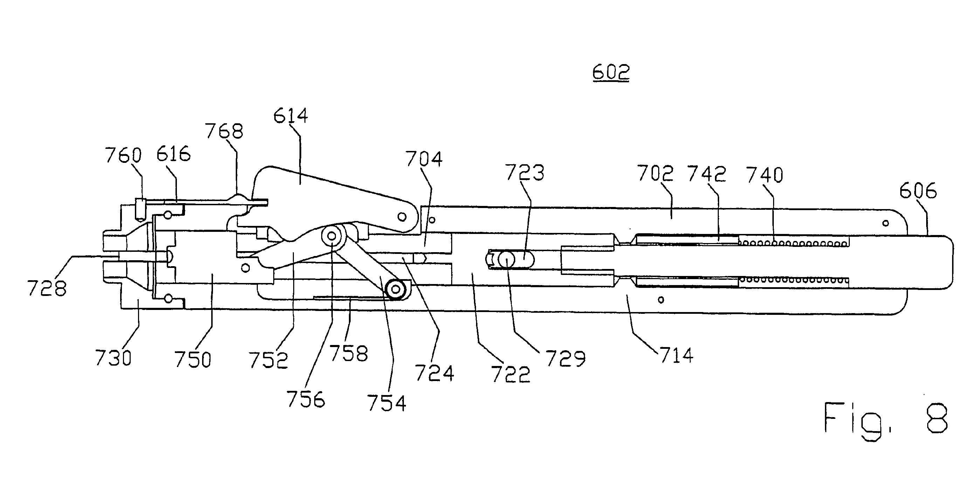

A more detailed view of the handpiece 602 is illustrated in FIGs. 7 and 8. In general, the handpiece 602 comprises three major systems, a housing system, an inserter attachment engagement system and an inserter attachment actuation system. The inserter attachment engagement system allows a user of the handpiece 602 to engage/disengage an inserter attachment to/from the handpiece. The inserter attachment actuation system functions to translate actuation of the lever 614 into movement of a pusher (not shown) at the tip 612 of the inserter attachment. Finally, the housing system serves to encase and substantially protect the inserter attachment engagement system and the inserter attachment actuation system. Note that, in a preferred embodiment, all of the components forming the handpiece 602 and inserter attachment 604 are made from sterilizable materials. Preferably, the constituent components of the handpiece 602 and inserter attachment 604 may be fabricated from any combination of the following materials: stainless steel, anodized aluminum, titanium, polysulfone, Radel® (polyethersulfone), silicone, epoxy or Buna-N. Generally, it is preferred that all components of the handpiece 602 and inserter attachment 604 be substantially free of sharp edges or corners, particularly any movable components or components that may come in contact with biological tissues. To this end, with respect to any metallic (e.g., stainless steel) components, it is preferred that such component be electropolished to minimize the generation of particulate matter that might otherwise result from the frictional engagement of various components. Although FIG. 7 and subsequent figures describe specific embodiments for the three major systems, those having ordinary skill in the art will recognize that various aspects of each system may be readily implemented using techniques other than those described in the instant specification.

As shown in FIG. 7, the housing system preferably comprises two semi-cylindrical members 702 that are mirror images of each other. The semi-cylindrical members 702 are preferably dimensioned to be suitable for handheld use. When coupled together, the semi-cylindrical members 702 define a longitudinal channel 704 within the housing 603 preferably centered upon a longitudinal axis 706. The longitudinal channel 704 has a substantially circular cross-sectional area and the exterior surfaces of the housing 603 are substantially cylindrical, although neither characteristic is a requirement and virtually any cross-sectional area and/or exterior surface shape may be equally employed. The longitudinal channel 704 extends through the length of the housing 603 and is accessible at both a first end 708 and second end 710 of the housing 603. Additionally, in a preferred embodiment, recesses 712 are formed in each semi-cylindrical member 702 such that an opening for the lever 614 is defined in a lateral surface of the housing 603. In practice, suitable fasteners, e.g., screws or rivets, may be used to couple the semi-cylindrical members 702 together.

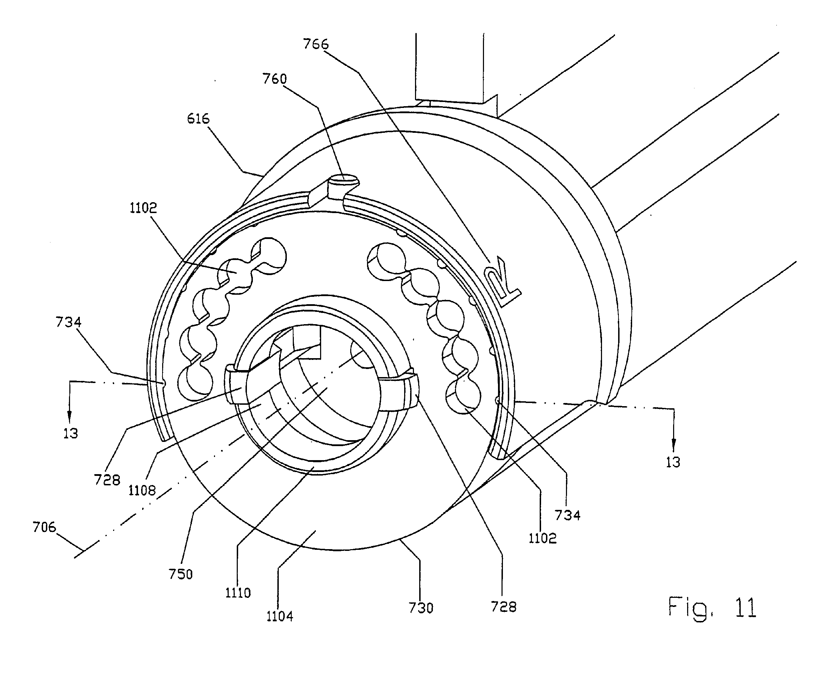

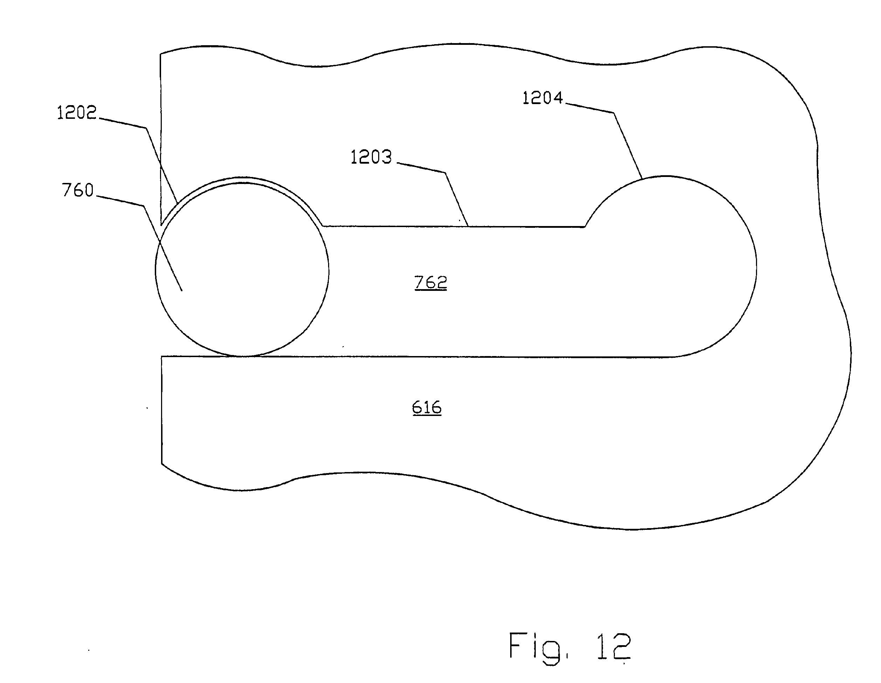

A first sliding member 720 is movably disposed within the longitudinal channel 704. In one embodiment, a stop 714 is provided integral to the semi-cylindrical members 702, thereby dividing the longitudinal channel 704. The first sliding member 720 is disposed within the longitudinal channel 704 between the stop 714 and the first end 708. As illustrated, the first sliding member 720 comprises a body 722. At least two legs 724 (preferably two, as shown) extend longitudinally from an end of the body 722 and are preferably parallel to each in a spaced apart relationship, i.e., substantially opposite each other. In one embodiment, each semi-cylindrical member 702 comprises at least one longitudinal groove 711 formed in an interior surface in which a corresponding leg is disposed in order to maintain the position of the leg in operation. Each leg 724 comprises an engaging surface 726 and terminates at a distal end in a finger portion 728. A portion of each leg 724, as well as their respective finger portions 728, extends through a first opening at the first end of the housing and a channel in a nose member 730 coupled to the first end of the housing. As described in greater detail below with particular reference to FIGs. 11 and 12, as the first sliding member 720 moves toward the nose member 730, the engaging surface 726 of each leg engages a biasing surface 732 of the nose member 730, thereby causing each leg and, more particularly, the finger portion 728 of each leg to be biased radially inward. In a preferred embodiment, the body 722 comprises a channel 723 extending through the body 722 and through which a fastener 729 may be passed. In this manner, the fastener 729, in addition to maintaining the semi-cylindrical members 702 coupled together, also serves to limit longitudinal movement of the first sliding member.

The first sliding member 720 is preferably biased to an initial, retracted position (i.e., at its furthest point of travel toward the stop 714) by a resilient member, such as a spring or other compressible component, disposed within the housing. In practice, the biasing resilient member may be directly interposed between the housing and the first sliding member 720 in such a way as to bias the first sliding member 720 to its initial position. In a presently preferred embodiment, however, such bias is applied via a button member 606 disposed within the longitudinal channel 704 between the stop 714 and the second end 710 of the housing. The button member 606 is coupled to the first sliding member 720 such that any force applied to the button member 606 is similarly applied to the first sliding member 720. Any of a variety of well known techniques may be used to couple the button member 606 to the first sliding member 720, with a threaded engagement being currently preferred. To apply the biasing force necessary to retain the first sliding member in its initial position, a first resilient member 740 engages the button member 606 and the housing. In the embodiment shown, the first resilient member 740 engages the housing via a spacer 742 positioned between the stop 714 and the resilient member 740. A suitable resilient member 740 is a compression spring, although other compressible components (such as a sleeve of compressible plastic) may be equally employed. The bias applied by the first resilient member 740 is of sufficient magnitude to maintain the first sliding member 720 at its initial position despite normal handling of the handpiece 602, but may be overcome by a countervailing force applied to the button member 606 (for example, by a user manually pressing the button member 606).

Regarding the inserter attachment actuation system, a second sliding member 750 is movably disposed within the longitudinal channel 704, preferably between the legs 724 of first sliding member 720. To accommodate such positioning, the second sliding member 750 preferably includes longitudinal grooves 751 (one shown) in which the legs 724 rest. As described in greater detail below, the second sliding member 750 comprises a surface (not shown) for engaging a pusher cap of an inserter attachment and, through movement of the second sliding member 750, causes a pusher in the inserter attachment to correspondingly move.

Movement of the second sliding member 750 is induced through a combination of the lever or trigger 614 and a linkage mechanism that converts radial movement of the lever 614 into translational (i.e., along the longitudinal channel 704) movement of the second sliding member. As known in the art, a wide variety of linkage mechanisms are available to convert radial motion into translational motion, many of which may be equally employed when implementing the present invention. A presently preferred linkage alternative is illustrated comprising a first link 752 coupled to the second sliding member 750 and a second link 754 that, in turn, is coupled to the housing. As illustrated, each link comprises complementary members to ensure stability of the resulting linkage and to provide a space for a roller 756 that provides a relatively low-friction mechanical contact between the linkage and the lever 614. A second resilient member 758 is maintained in a fixed relationship relative to the linkage and the housing so as to bias the lever 614, via the linkage, to a fully extended position. In the embodiment shown, the second resilient member 758 comprises a torsion spring that flexes against the second link 754.

Finally, the slidable trigger lock 616 is illustrated in greater detail. In particular, the slidable trigger lock 616 comprises a compression fit sleeve having a circumferential length spanning an arc within the approximate range of 190 to 359 degrees, preferably within the range of 345 to 355 degrees, and overlying the outer circumference of the nose member 730 and housing. At least one ridge 768 is provided on an exterior surface of the trigger lock 616 that allows a user of the handpiece 602 to feel for and manipulate the trigger lock 616 using a single finger (e.g., the user's middle finger) without having to look directly at the handpiece. The dimensions and positioning of the at least one ridge 768 may be selected to optimize such use as a matter of design choice. The compression fit of the trigger lock 616 around the nose member 730 and housing allows the trigger lock 616 to be moved longitudinally along the outer surface of the nose member 730 and housing. A stop pin 760 mounted in the nose member and in contact with a groove 762 in the trigger lock 616 limits longitudinal movement away from the housing, as well as rotational movement, whereas longitudinal movement toward the housing is limited by contact of the trigger lock 616 with the lever 614. Furthermore, the trigger lock 616, when positioned in contact with the lever 614, engages a notch 764 in the lever 614 such that movement of the lever 614 is substantially prevented. Conversely, engagement of the groove 762 in the trigger lock 616 with the stop pin 760 causes the trigger lock 616 to disengage from the notch 764, thereby allowing free movement of the lever 614. In one embodiment, an indicator (e.g., a colored band or other surface marking; not shown) is provided on the lateral surface of the housing 702 such that, when the trigger lock 616 fully engages the notch 764, the indicator is covered by the trigger lock 616 indicating that the handpiece 602 is not "armed" (i.e., not capable of moving the first sliding member). When the trigger lock 616 fully engages the stop pin 760, and disengages from the notch 764, the trigger lock 616 does not cover the indicator thereby indicating that the handpiece 602 is now armed (i.e., capable of moving the first sliding member). This visible indication of the status of the handpiece 602 helps prevent inadvertent operation of the handpiece 602 and, potentially, inadvertent discharge of the implant from the implant attachment. While the trigger lock 616 as described herein provides a simple mechanism for preventing movement of the lever 614, those having ordinary skill in the art will appreciate that other mechanisms may be employed to prevent movement of the lever 614, the linkage or the second sliding member 750.

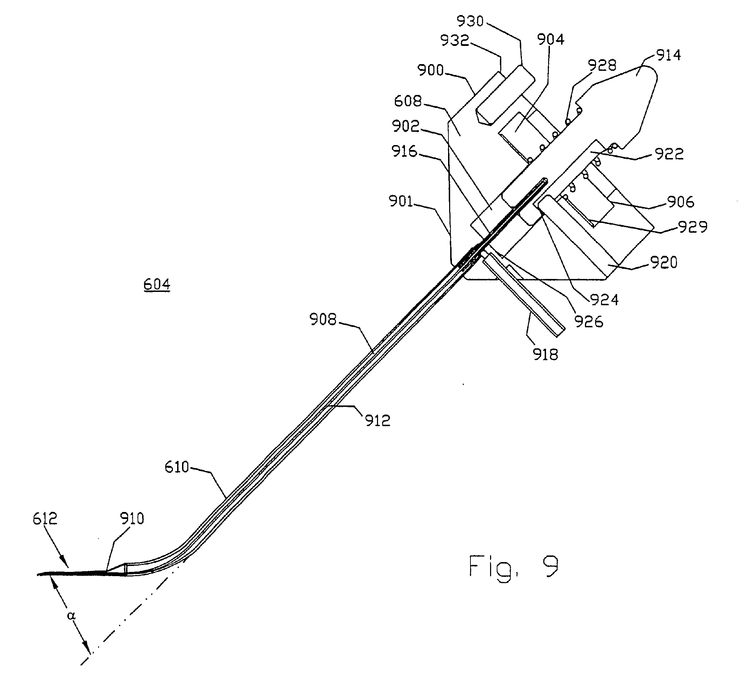

Referring now to FIG. 9, a cross-sectional side view of an inserter attachment 604 having particular use for implanting retinal implants is shown. The inserter attachment 604 comprises a body member 608 preferably formed having a cylindrical base portion 900, a conical portion 901 and having a longitudinal channel 902 formed therein. Within the conical portion 901, the longitudinal channel 902 has a relatively narrow cross-sectional area. Preferably, within the cylindrical portion 900, the longitudinal channel 902 has a substantially wider cross-sectional area 904 to accommodate insertion of the handpiece nose member 730 and fingers 728. Additionally, near the base of the cylindrical portion 900, a retention surface 906 is provided for engaging the finger portions 728 of the first sliding member 720 when the inserter attachment 604 is coupled to the handpiece 602, as described in greater detail below, particularly with reference to FIGs. 11 and 12.

As further shown in FIG. 9, the inserter attachment 604 includes a conduit 610 coupled to the body member 608 at a proximal end of the conduit such that the interior passage 908 of the conduit is in communication with the longitudinal channel 902. The conduit 610 is of sufficient length to allow the inserter tip 612 to be inserted intraocularly and positioned in close proximity to the retina, or even sub-retinally, when being manipulated externally via the handpiece. In a presently preferred embodiment, the distal end of the conduit terminates in a substantially flattened portion 910 to facilitate intraocular and sub-retinal insertion of the inserter tip 612. To better match the curvature of the eye, where the inserter attachment 604 is to be used for intraocular applications, the conduit 610 is curved near the distal end of the conduit. The curvature of the conduit 610 is such that the flattened portion 910 is at an angle, α, in the range of 0 to about 135 degrees. The particular angle depends on the application. In the case of retinal implants, the placement of the implant, the entry point into the eye and the hand preference of the surgeon will all contribute to the particular angle employed. For instance, where a retinal implant is to be placed in the temporal region of the posterior hemisphere of the right eye through a temporal sclerotomy by a right-handed surgeon (assuming that the surgeon is positioned above the head of the supine patient), an angle of approximately 45 degrees may be employed. For a temporal, posterior placement through a nasal sclerotomy by a left-handed surgeon, an angle of approximately 135 may be appropriate.

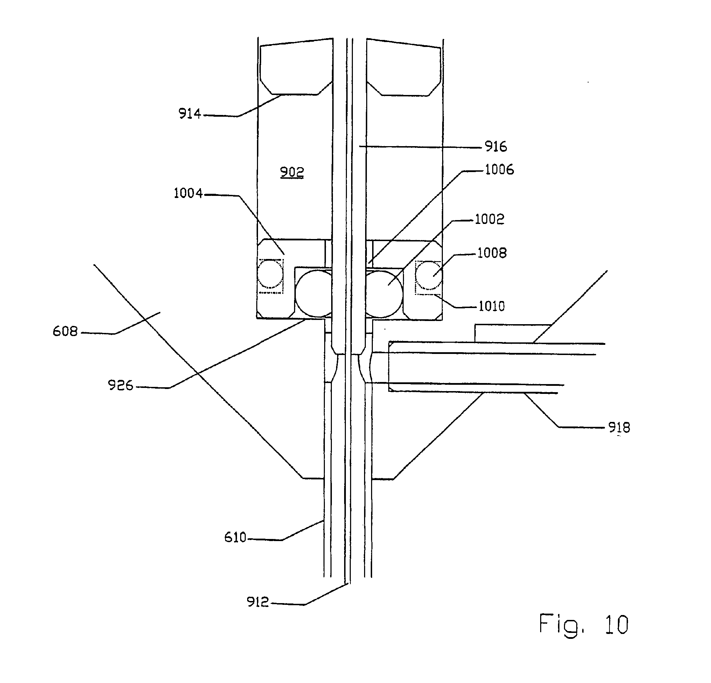

As described in greater detail below, the inserter tip 612 comprises a pusher used to express a retinal implant from the inserter tip, which pusher is controlled through actuation of the second sliding member 750 via the lever 614 within the handpiece. To transfer movement of the second sliding member 750 to the pusher, a linkage 912 is disposed within the conduit 610 and the body member 608. The linkage 912 is coupled to a pusher cap 914 within the body member 608, preferably via a rigid extension 916. In a preferred embodiment, the linkage 912 comprises a wire having sufficient stiffness to resist bending or kinking when a translational force is applied to the pusher cap 914, but is sufficiently compliant as to be easily installed in the conduit 610. Furthermore, the linkage preferably has a cross-sectional area less than the cross-sectional area of the interior passage 908, thereby enabling fluid flow through the conduit. For example, in a presently preferred embodiment, a substantially rectangular cross-sectional wire having a cross-sectional area of approximately 0.000039 square inches (0.025161 square millimeters) is provided in a substantially circular cross-sectional conduit having a cross-sectional area of approximately 0.00053 square inches (0.341935 square millimeters). To facilitate fluid flow, a port 918, radially mounted within the body member 608, is provided in fluid communication with the conduit. In practice, the port 918 may be coupled to a fluid or vacuum source as needed. In an alternate embodiment, further illustrated in FIG. 10, a seal member 1002 may be provided to prevent backflow of fluids into the channel 902 and, possibly, into the handpiece 602. As illustrated in FIG. 10, the seal member 1002 preferably comprises an O-ring fabricated from a compliant material such as silicone, Buna-N or other medical grade elastomers and positioned within the channel 902 and near a bottom surface 926 thereof. The seal member 1002 is dimensioned such that it provides a substantially fluid-tight seal around the extension 916. The seal member 1002 is preferably maintained in its position near the bottom 926 via a retention cap 1004 having a passage 1006 through which the extension 916 passes. The retention cap 1004 is dimensioned such that it is in contact with the seal member 1002 so as to provide a fluid-tight seal. Similarly, the retention cap 1004 provides a fluid-tight seal between itself and the surface of the channel 902. In an alternative arrangement, illustrated by the dashed lines, an additional seal member 1008 (which may also comprise, for example, an O-ring as described above) is disposed in an annular recess 1010 formed in the retention cap 1004 to thereby provide a fluid-tight seal around the periphery of the retention cap 1004. While a particular seal member arrangement is illustrated in FIG. 10, those having ordinary skill in the art will appreciate that other schemes may be equally employed to prevent the back flow of fluids.

Referring again to FIG. 9, the pusher cap 914 is snugly but slidably disposed with the longitudinal channel 902. Grooves (not shown) formed in the pusher cap 914 provide air vents to prevent air trapping. A stop pin 920, radially mounted through the body member 608, cooperates with a retention groove 922 formed in the pusher cap 914. In particular, a retention surface 924 of the retention groove 922, in cooperation with the stop pin 920, limits outward travel of the pusher cap 914 and further retains the pusher cap within the body member 608. The bottom 926 of the channel 902 (or, in the case of the alternate embodiment described above relative to FIG. 10, the retention cap 1004) limits inward travel of the pusher cap 914 and, consequently, limits travel of the pusher within the inserter tip 612. In this manner, a user of the handpiece 602 and inserter attachment is substantially prevented from overextending the pusher and potentially causing injury to delicate tissues.

In order to maintain the pusher in a substantially retracted position (absent a competing force provided by the handpiece), a third resilient member 928, such as a compression spring or other compressible material, is disposed between the pusher cap 914 and the body member 608. The force exerted by the third resilient member 928 is transferred to the pusher via the pusher cap 914 and the linkage 912 to maintain the pusher in its resting, retracted position. A competing force may be applied to the pusher cap via, for example, the second sliding member 750 of the handpiece 602 to overcome the biasing force of the third resilient member 928 thereby moving the pusher. A fourth resilient member 929 may be optionally positioned at the base of the wider cross-sectional portion 904 of the channel to prevent partial or improper coupling of the inserter apparatus 604 to the handpiece 602. In one embodiment, the fourth resilient member 929 may comprise an open ring having a flexure or bend such that the ring does not lie flat (not shown) on the base of the channel 904 and thereby forms a compression spring. When the handpiece 602 is brought into contact with the inserter attachment 604, the nose member 730 contacts the fourth resilient member 929. The repulsive bias provided by the fourth resilient member 929 is overcome as the nose member 730 is fully inserted into the channel 904. In the event that the finger portions 728 fail to fully engage the retention surface 906, the repulsive bias provided by the fourth resilient member 929 prevents partial engagement (and thus the potential for subsequent, unexpected decoupling) by forcing the inserter attachment 604 away from the nose member 730.

Finally, a locating pin 930 is mounted in a perpendicularly projecting fashion on a rear-facing surface 932 of the body member 608. As described in greater detail below, the locating pin 930 cooperates with recesses formed in the nose member 730 to maintain the inserter attachment 604 at a fixed alignment relative to the handpiece. Although a pin is illustrated for this purpose, it is understood that virtually any type of projecting member may be used and the present invention is not limited in this regard. In one embodiment, the body member 608 can be fabricated from a substantially transparent or translucent material such that the portion of the locating pin 930 disposed within the body member 608 remains visible. This would permit more accurate alignment of the locating pin 930 with recesses disposed in the nose member 730 when courting the inserter attachment 604 to the handpiece 602.

Referring now to FIG. 11, a more detailed view of a presently preferred embodiment of the nose member 730 is provided. In particular, the nose member 730 is seen to comprise at least two recesses 1102 formed in a forward-facing surface 1104 perpendicular to the longitudinal axis 706 of the handpiece 602. The nose member is substantially centered upon the longitudinal axis 706, and the recesses 1102 are preferably arranged at various angular positions about the longitudinal axis 706. In one embodiment, the recesses 1102 are placed at selected angles so as to accommodate user handling preferences of the handpiece 602. For example, in a presently preferred embodiment, the recesses 1102 are placed at ±26°, ±43°, ±60°, ±77° and ±94° angles relative to a 12 o'clock position when facing the forward-facing surface 1104. A greater or lesser number of recesses 1102 may be provided at the same or different angles as a matter of design choice. By placing the recesses 1102 at various angular positions, the locating pin 930 of the inserter attachment 604 can mate with one of the recesses 1002 to maintain the inserter attachment 604 at a selected angular alignment. Those having ordinary skill in the art will appreciate other mechanisms for maintaining alignment of the inserter attachment may be equally employed. For example, alternative mating mechanisms could be provided on other surfaces of the nose member, e.g., longitudinal grooves placed at various angular positions on an internal circumferential surface 1108 of the nose member 730. Further still, it is understood that the placement of the cooperating members could be reversed, e.g., the recesses 1102 could be provided on the rear-facing surface 932 of the inserter attachment 604 and the locating pin 920 could be provided on the forward-facing surface 1104 of the nose member 730.

FIG. 11 illustrates the various indicia that may be provided to assist a user when coupling and aligning the inserter attachment with the handpiece 602. In particular, the nose member 730 may include grooves 734 (see also FIG. 7) formed in an outer circumferential surface of the nose member and angularly aligned with corresponding ones of the recesses 1102. One or more of the grooves 734 can be highlighted or otherwise made visually (or even tactilely) distinctive relative to the other grooves to indicate a nominal or suggested alignment. Additionally, the slidable trigger lock 616 may include further indicia 766 (one shown) indicating the orientation (i.e., left or right hand) of the recesses 1102 aligned with the indicia 766.

FIG. 11 also illustrates the manner in which the stop pin 760 engages the groove 762 formed in the slidable trigger lock 616. In the embodiment shown, the lateral surfaces of the groove 762 are uniformly dimensioned along its longitudinal axis to conform to the diameter of the stop pin 760. In an alternative embodiment, illustrated in FIG. 12, at least a portion of the lateral surfaces 1202-1204 of the groove 762 are non-uniform relative to the diameter of the stop pin 760. As shown in the embodiment of FIG. 12, the lateral surfaces of the groove 762 comprise a first rounded surface 1202 and a second rounded surface 1204, wherein each rounded surface 1202, 1204 has a radius substantially similar to a radius of the stop pin 760. A straight surface 1203 spans the distance between the first and second rounder surfaces 1202, 1204. The width of the groove 762 defined by the straight surface 1203 is preferably less than the diameter of the stop pin 760. As a result of this configuration, movement of the slidable trigger lock 616 and the varying widths of the groove provided by the rounded surfaces 1202, 1204 and the straight surface 1203 collectively cause the stop pin 760 to traverse the groove 762 in an abrupt fashion giving rise to an audible report (i.e., a "click" sound) when the stop pin 760 comes to rest in a portion of the groove 762 defined by either of the rounded surfaces 1202, 1204. In this manner, a user of the handpiece is provided an audible confirmation that the slidable trigger lock 616 has been fully moved thereby arming or disarming the handpiece. Those having ordinary skill in the art will appreciate that other, similar arrangements could be provided for this purpose. For example, rather than providing rounded surfaces on only one side of the groove, as shown in FIG. 12, such rounded surfaces could be provided on both sides of the groove as a matter of design choice.

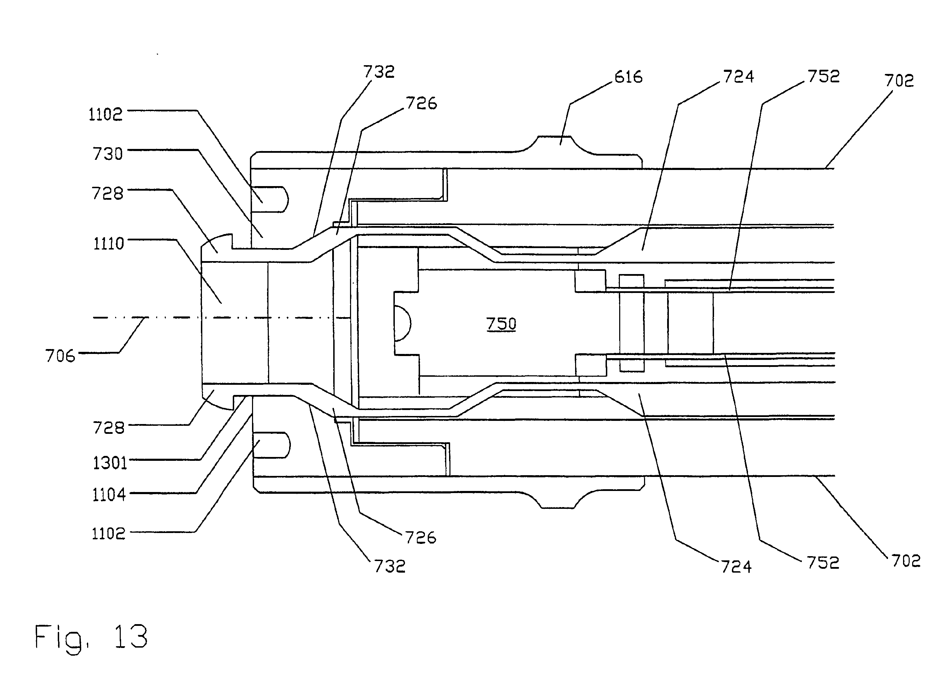

Finally, FIG. 11 illustrates a raised annular portion 1110 of the nose member 730. When coupled to the inserter attachment 604, the raised annular portion 1110 projects into the wider cross-sectional portion 904 of the longitudinal channel 902 of the inserter attachment 604. Furthermore, the raised annular portion 1110 includes notches to accommodate the legs 724 and finger portions 728 of the first sliding member 720 and, more particularly, to accommodate protrusion of the finger portions 728 beyond an outer circumference 1201 of the raised annular portion 1110, as further illustrated in FIG. 13.

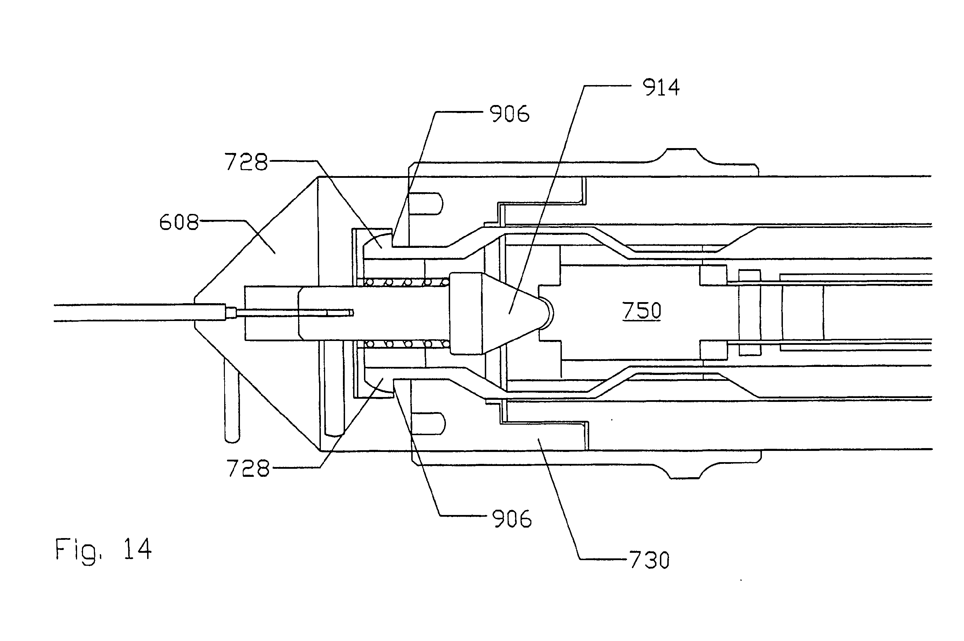

Referring now to FIG. 13, a cross-sectional view taken along line 13-13 of FIG. 11 is shown. The nose member 730 includes a biasing surface 732 that defines a portion of a longitudinal channel through the nose member 730. In particular, the biasing surface 732 is a beveled surface that substantially contacts a similarly beveled engaging surface 726 of the legs 724 when the first sliding member 720 moves along the longitudinal channel 704 toward the nose member 730. While in the retracted position, the finger portions 728 protrude beyond the outer circumference 1301 of the raised annular portion 1110. The material employed to fashion the legs 724 preferably has sufficient stiffness to ensure the protrusion of the finger portions 728 beyond the outer circumference 1301 while in the retracted position. As the first sliding member 720 is moved forward (i.e., in the direction of the nose member 730), the engagement of the biasing surface 732 and the engaging surfaces 726 cause the legs 724 and, consequently, the finger portions 728 to be biased radially inward. As a result, the finger portions 728 move both axially forward and radially inward as indicated by the arrows. In so doing, the distance to which the finger portions 728 protrude beyond the outer circumference 1301 is reduced (possibly even to the point of being entirely within the outer circumference 1301) such that the body member 608 of the inserter attachment 604 may snugly engage the nose member 730. This is further illustrated in FIG. 14 where the body member 608 is coupled to the nose member 730. The first sliding member 720 has been returned to its retracted position and, consequently, the finger portions 728 once again protrude beyond the outer circumference 1301 of the raised annular portion 1110. As a result, the finger portions 728 now engage the retention surface 906 thereby securely maintaining the body member 608 in a fixed relationship with the handpiece 602. FIG. 14 also illustrates the manner in which the pusher cap 914 engages the second sliding member 750.

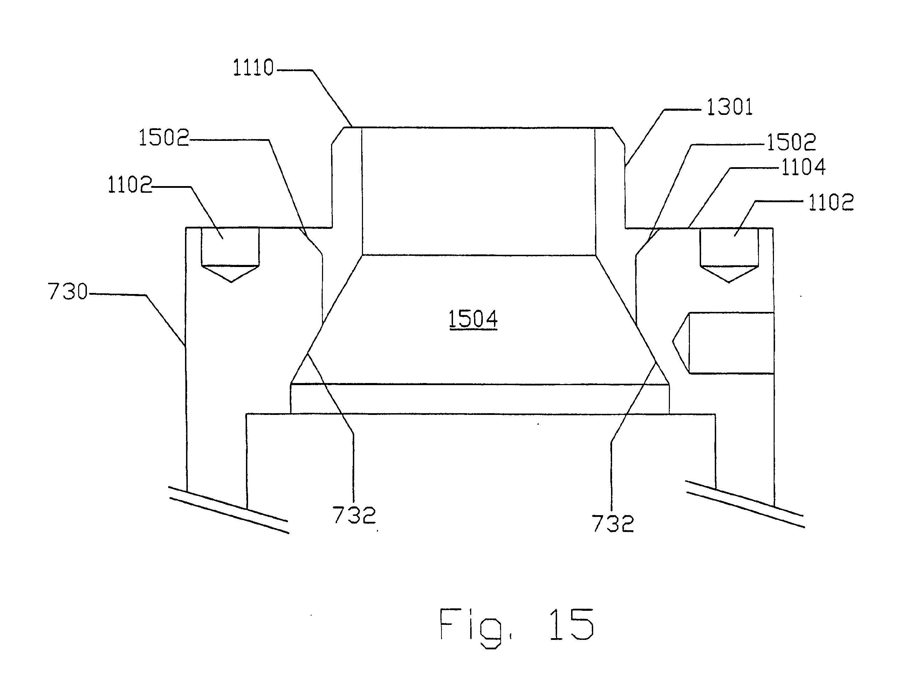

In an alternate embodiment of the present invention, illustrated in FIG. 15, it is desirable to have the initial, retracted position of the first sliding member 720 such that the finger portions 728 are positioned within a channel 1504 of the nose member 730, rather than protruding beyond the outer circumference 1201 of the raised annular portion 1110. To this end, a beveled outer surface 1502 is provided as shown. The radial width of the beveled outer surface 1502 is preferably wider than the length to which the finger portions 728 extend radially outward. In this manner, as the first sliding member 720 travels back to its initial, retracted position (by virtue, for example, of the bias provided by the first resilient member 740; see FIG. 7), the finger portions 728 engage the beveled outer surface 1502 and are thereby prevented from engaging or otherwise catching on the forward-facing surface 1104 of the nose member 730. As a result, the finger portions 728 are positioned within the channel 1404 when the first sliding member 720 is in its initial, retracted position.

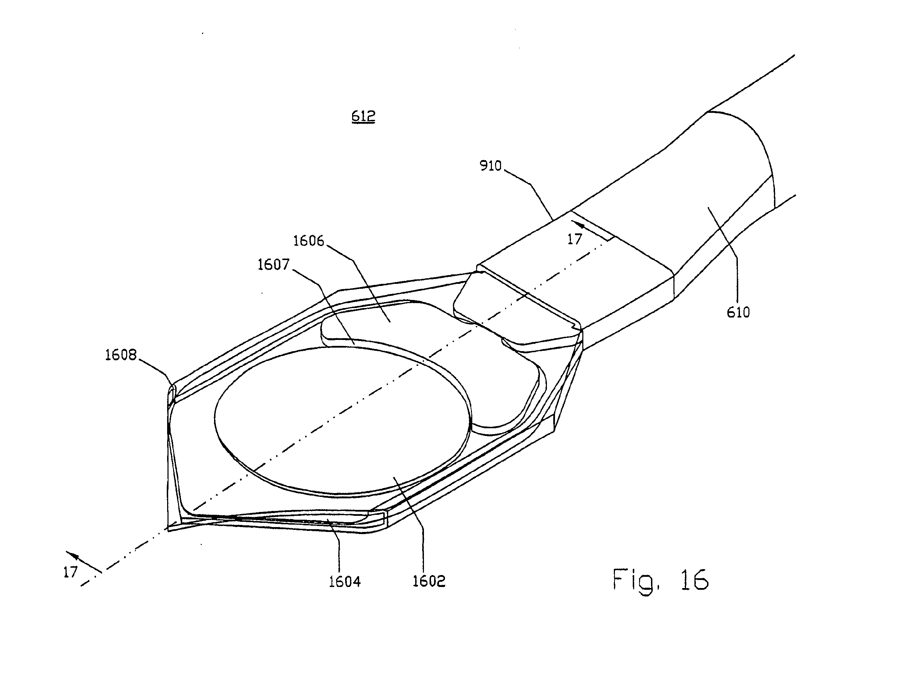

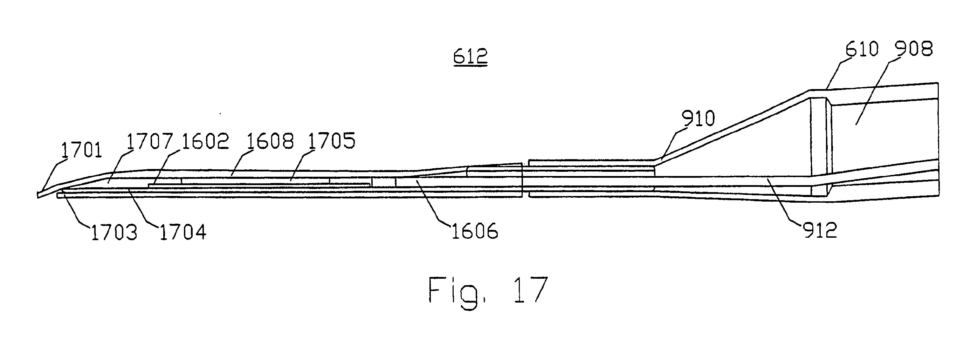

Referring now to FIGs. 16 and 17, the inserter tip 612 is illustrated in greater detail. The inserter tip 612 functions as a holder for the implant 1602 as well as a delivery mechanism when the implant is deposited at the implant site. The inserter tip 612 comprises an open-ended tray 1604 connected to the distal end of the conduit 610. Preferably, the tray 1604 is dimensioned so at to contain the implant 1602 and provide sufficient clearance as to allow fluid flow around the implant. As noted above, it is preferred that the distal end of the conduit 610 comprise a flattened portion 910 so as to minimize the profile of the inserter tip 612. The tray 1604 is preferably formed to have a tapered or pointed tip at its open end to facilitate surgical insertion of the inserter tip 612. It is understood, however, that the open end of the tray 1604 may comprise other shapes, such as a semicircular shape, as a matter of design choice. Preferably, in order to avoid snagging of ocular tissues, all corners of the tray 1604 are rounded (i.e., have a radius).

The linkage 912 disposed within the conduit 610 terminates in or is otherwise coupled to a pusher 1606 that rests within the tray 1604. The tray 1604 is dimensioned to allow the pusher 1606 to travel freely along the length of the tray 1604 when actuated by operation of the handpiece 602, as described above. The pusher 1606 preferably comprises a surface 1607 for conformally engaging the implant 1602. In the example shown, the surface 1607 is curved to match the substantially circular shape of the implant. Of course, implants may comprise other shapes thereby requiring pushers of similarly different shapes.

A feature of the present invention is the use of a cover 1608 that substantially envelopes the tray 1604, pusher 1606 and conduit 610. The cover 1608 substantially envelopes and thereby smoothes out any surfaces that might provide an opportunity for catching or snagging tissues. Generally, the cover 1608 may be fashioned from any of a number of well known polymers and, in a preferred embodiment, is fashioned from a substantially transparent polymer to allow visual observation of the implant 1602 throughout usage of the inserter tip 612. In combination with the tray 1604 (particularly the side walls 1705 of the tray 1604; see FIG. 17), the cover 1608 defines a space 1707 in which the implant 1602 may be safely positioned. In a preferred embodiment, the cover 1608 is formed of a material that substantially retains its formed shape (i.e., a memory material), but that is relatively compliant, such as so-called shrink tubing. The cover 1608 is effectively an additional conduit that envelops the tray 1604, pusher 1606 and conduit 610 and that has one end (i.e., the end nearest to the body 608; not shown) substantially closed around the conduit 610 proximate the body 608. Referring to FIG. 17, at least two flaps 1701, 1703 are preferably fashioned out of the conduit forming the cover 1608 at an open end thereof (i.e., the end farthest from the body 608). The flaps 1701, 1703 form a pinched arrangement such that the space 1707 in which the implant 1602 resides is at least partially closed by the flaps 1701, 1703. In this manner, the flaps 1701, 1703 help retain the implant within the space 1707.

Preferably, the flap 1701, 1703 are tapered in a manner similar to the tray 1604, i.e., to a point, although this is not a requirement. Indeed, one or more of the flaps 1701, 1703 may comprise a shape other than a point, e.g., a semicircle, etc. In one embodiment, the angle subtended by the point on each flap is approximately 100°. In those instances in which one or more flaps do taper to a point, the angle subtended by the point on each flap may be equal to each other or they may be different and, furthermore, may be equivalent to or different from the angle subtended by the point of the tray 1604. In a presently preferred embodiment, the angle of the point of the lower flap is substantially the same as the angle of point of the tray, and the angle of the upper flap is dictated by the overlap of the upper flap over the lower flap.

In one embodiment of the present invention, one of the flaps 1701 is longer than the other flaps 1703 and overlays the other flaps 1703 (as well as the tray 1604) such that only the single leading edge of the longer flap 1701 is presented. By covering up the leading edges of the other flaps 1703 (as well as the leading edge of the tray 1604) in this manner, the likelihood of snagging delicate tissues, such as retinal tissues, is minimized. Note that an "overbite" arrangement, i.e., in which the upper flap 1701 of two flaps overlies the lower flap 1703, is illustrated in FIGs. 16 and 17. In practice, an "underbite" arrangement, i.e., in which the lower flap 1703 of two flaps overlies the upper flap 1701, may be equally employed.

The present invention as described above may be of particular benefit when used to deploy retinal, especially sub-retinal, implants. In these cases, an incision (a sclerotomy) is made in the sclera of the eye and, in the case of a sub-retinal implant, an opening is also made in the retina (a retinotomy). Using a inserter attachment 604 coupled as described above to a handpiece 602, the inserter tip 612, comprising the implant 1602, is thereafter inserted through the sclerotomy and, in the case of a sub-retinal implant, optionally through the retinotomy. Once intraocularly inserted, the pusher 1606 may be controlled, as described above, to deposit the retinal implant 1602 as desired. In this manner, a much greater degree of control is provided when implanting the retinal implant than was previously provided using prior art techniques.

It is additionally understood that the present invention, particularly the handpiece portion thereof, may be used in any context in which it would be advantageous to provide a translational movement to an attachment. Stated another way, the inserter attachment may comprise any device that would benefit from the application of a pushing or translational movement. For example, various types of attachments could be devised that convert the translational movement of the second sliding member 750 into other types of movement, such as rotational, radial, pinching, cutting, etc.

It is intended that foregoing detailed description should be regarded as illustrative rather than limiting, and that it be understood that the following claims, including all equivalents are intended to define the scope of this invention.

## Claims

1. An instrument (600) for use with a medical implant, comprising:
a housing (603) comprising a first end (708), a second end (710) and a longitudinal channel (704) defined therein between the first end (708) and the second end (710), the first end (708) comprising a first opening;
a nose member (730), coupled to the first end of the housing (603), comprising a nose channel and a biasing surface (732);
a first sliding member (720), movably disposed within the longitudinal channel (704) and the nose channel through the first opening, comprising a body (722) and at least two legs (724) longitudinally disposed at an end of the body (722), each leg comprising.an engaging surface (726) and terminating in a finger portion (728) at a distal end relative to the body (722); and
an inserter attachment (604) for use in inserting a medical implant into a patient's body, the inserter attachment (604) being adapted to be coupled to the nose member (730) via the finger portion (728) of each leg (724),
wherein movement of the first sliding member (720) along the longitudinal channel (704) causes the engaging surface (726) of each leg of the at least two legs (724) to engage the biasing surface (732) and thereby to bias the finger portion (728) of each leg (724) inward, and
wherein the finger portion (728) of each leg (724) is adapted to engage a surface of the inserter attachment (604) to maintain the inserter attachment (604) in a fixed relationship relative to the nose member (730) when the first sliding member (720) is at an initial position.

2. An instrument (600) according to claim 1, wherein a button member (606) is provided operatively connected to the first sliding member (720) to allow user-actuated movement of the first sliding member (720), and a first resilient member (740) engages the button member (606) and the housing (603) to apply a biasing force to retain the first sliding member (720) in its initial position.

3. An instrument (600) according to claim 1, the nose member (730) comprising a surface having at least two recesses (1102) defined therein,
wherein the at least two recesses (1102) are positioned at different angular positions relative to a longitudinal axis (706) of the housing (603) such that a projecting member (930) of the inserter attachment can mate with one of the at least two recesses (1102) thereby to maintain the inserter attachment(604) at a selected alignment.

4. An instrument (600) according to any one of claims 1 to 3, further comprising:
a retinal implant (1602) disposed within the inserter attachment (604).

5. An instrument (600) according to any one of claims 1 to 4, wherein the inserter attachment (604) comprises:
a body member (608) having a longitudinal channel (902) defined therein;
a conduit (610) coupled to and in communication with the longitudinal channel (902) of the body member (608) at a proximal end of the conduit (610);
a linkage (912), disposed within the conduit (610) and the longitudinal channel (902), terminating in a pusher cap (914) at a first end of the linkage (912) proximal the body member (608) and terminating in a pusher (1606) at a second end of the linkage (912) at a distal end of the conduit (610); and
a resilient member (928) interposed between the body member (608) and the pusher cap (914) such that the pusher (1606) is biased into a retracted position.

6. An instrument (600) according to claim 5,
wherein a lever (614) and linkage arrangement is provided to impart longitudinal or axial movement to a second sliding member (750) disposed within the housing (603), and
wherein the pusher cap (914), when the inserter attachment (604) is coupled to the nose member (730), engages the second sliding member (750) such that longitudinal movement of the second sliding member (750) within the housing is translated, via the conduit linkage (912) of the inserter attachment (604), to movement of the pusher (1606) at the distal end of the conduit (610).

7. An instrument (600) according to claim 5 or claim 6, wherein the inserter attachment (604) further comprises:
a projecting member (930) coupled to the body member (608), wherein the projecting member (930) is adapted to mate with at least one recess (1102) disposed in the instrument (600) to maintain alignment of the inserter attachment (604).

8. An instrument (600) according to claim 5 or claim 6, wherein an open-ended tray (1604) is coupled to the distal end of the conduit (610) such that the pusher (1606) rests in the open-ended tray (1604), and wherein the open-ended tray (1604) comprises a space for holding the medical implant to be inserted into the patient's body.

9. An instrument (600) according to any one of claims 1 to 4, wherein the inserter attachment (604) comprises:
a body member (608) having a longitudinal channel (902) defined therein;
a conduit (610) coupled to and in communication with the longitudinal channel (902) of the body member (608) at a proximal end of the conduit (610);
an open-ended tray (1604) coupled to a distal end of the conduit (610); and
a cover (1608), enveloping the open-ended tray (1604), having an open end opposite the conduit (610), the open end comprising at least two flaps (1701, 1703) in which one of the at least two flaps (1701, 1703) is longer than and substantially overlies the other of the at least two flaps (1701, 1703) such that a space defined in part by the open-ended tray (1604) and the cover (1608) is at least partially closed by the at least two flaps (1701, 1703).

10. An instrument (600) according to claim 9, wherein the inserter attachment further comprises:
a retinal implant (1602) disposed within the space.

## Patentansprüche

1. Instrument (600) zur Verwendung mit einem medizinischen Implantat, aufweisend:
ein Gehäuse (603) mit einem ersten Ende (708), einem zweiten Ende (710) und einem darin zwischen dem ersten Ende (708) und dem zweiten Ende (710) definierten Längskanal (704), wobei das erste Ende (708) eine erste Öffnung aufweist;
ein Nasenelement (730), das mit dem ersten Ende des Gehäuses (603) gekoppelt ist und einen Nasenkanal und eine Vorspannfläche (732) aufweist;
ein erstes Gleitelement (720), das im Längskanal (704) und im Nasenkanal durch die erste Öffnung beweglich angeordnet ist und einen Körper (722) und mindestens zwei an einem Ende des Körpers (722) längs angeordnete Beine (724) aufweist, wobei jedes Bein eine Eingriffsfläche (726) aufweist und in einem Fingerabschnitt (728) an einem fernen Ende relativ zum Körper (722) endet; und
einen Einführervorsatz (604) zur Verwendung bei Einführen eines medizinischen Implantats in den Körper eines Patienten, wobei der Einführervorsatz (604) so ausgeführt ist, dass er über den Fingerabschnitt (728) jedes Beins (724) mit dem Nasenelement (730) gekoppelt werden kann,
wobei eine Bewegung des ersten Gleitelements (720) entlang des Längskanals (704) bewirkt, dass die Eingriffsfläche (726) jedes Beins der mindestens zwei Beine (724) mit der Vorspannfläche in Eingriff kommt (732) und **dadurch** den Fingerabschnitt (728) jedes Beins (724) nach innen vorspannt, und
wobei der Fingerabschnitt (728) jedes Beins (724) so ausgeführt ist, dass er eine Fläche des Einführervorsatzes (604) in Eingriff nimmt, um den Einführervorsatz (604) in einer festen Beziehung relativ zum Nasenelement (730) zu halten, wenn sich das erste Gleitelement (720) in einer Anfangsposition befindet.

2. Instrument (600) nach Anspruch 1, bei dem ein Knopfelement (606) angeordnet ist und mit dem ersten Gleitelement (720) funktional verbunden ist, um eine benutzerbetätigte Bewegung des ersten Gleitelements (720) zu ermöglichen, und ein erstes elastisches Element (740) das Knopfelement (606) und das Gehäuse (603) in Eingriff nimmt, um eine Vorspannungskraft auszuüben, um das erste Gleitelement (720) in seiner Anfangsposition zu halten.

3. Instrument (600) nach Anspruch 1, wobei das Nasenelement (730) eine Fläche aufweist, in der mindestens zwei Aussparungen (1102) definiert sind,
wobei die mindestens zwei Aussparungen (1102) in verschiedenen Winkelpositionen relativ zur Längsachse (706) des Gehäuses (603) angeordnet sind, so dass sich ein hervorstehendes Element (930) des Einführervorsatzes mit einem der mindestens zwei Aussparungen (1102) paaren kann, um **dadurch** den Einführervorsatz (604) in einer gewählten Ausrichtung zu halten.

4. Instrument (600) nach einem der Ansprüche 1 bis 3, ferner aufweisend:
ein im Einführervorsatz (604) angeordnetes Netzhautimplantat (1602).

5. Instrument (600) nach einem der Ansprüche 1 bis 4, wobei der Einführervorsatz (604) aufweist:
ein Körperelement (608), in dem ein Längskanal (902) definiert ist;
ein Rohr (610), das an einem nahen Ende des Rohrs (610) mit dem Längskanal (902) des Körperelements (608) gekoppelt ist und mit ihm in Verbindung steht;
ein Gestänge (912), das im Rohr (610) und dem Längskanal (902) angeordnet ist und in einer Schieberkappe (914) an einem ersten, zum Körperelement (608) nahen Ende des Gestänges (912) endet und in einem Schieber (1606) an einem zweiten Ende des Gestänges (912) an einem fernen Ende des Rohrs (610) endet; und
ein elastisches Element (928), das derart zwischen dem Körperelement (608) und der Schieberkappe (914) angeordnet ist, dass der Schieber (1606) in eine zurückgezogene Position hinein vorgespannt ist.

6. Instrument (600) nach Anspruch 5,
wobei eine Hebel- (614) und Gestängeanordnung vorgesehen ist, um ein im Gehäuse (603) angeordnetes zweites Gleitelement (750) in eine Längs- oder Axialbewegung zu versetzen, und
wobei die Schieberkappe (914) bei mit dem Nasenelement (730) gekoppeltem Einführervorsatz (604) das zweite Gleitelement (750) in Eingriff nimmt, so dass eine Längsbewegung des zweiten Gleitelements (750) im Gehäuse über das Rohrgestänge (912) des Einführervorsatzes (604) in eine Bewegung des Schiebers (1606) am fernen Ende des Rohrs (610) umgesetzt wird.

7. Instrument (600) nach Anspruch 5 oder Anspruch 6, wobei der Einführervorsatz (604) ferner aufweist:
ein mit dem Körperelement (608) gekoppeltes hervorstehendes Element (930), wobei das hervorstehende Element (930) so ausgeführt ist, dass es sich mit mindestens einer im Instrument (600) angeordneten Aussparung (1102) paart, um die Ausrichtung des Einführervorsatzes (604) aufrechtzuerhalten.

8. Instrument (600) nach Anspruch 5 oder Anspruch 6, wobei ein offenendiges Tablett (1604) mit dem fernen Ende des Rohrs (610) gekoppelt ist, so dass der Schieber (1606) im offenendigen Tablett (1604) ruht, und wobei das offenendige Tablett (1604) einen Raum zur Aufnahme des medizinischen Implantats, das in den Körper des Patienten eingeführt werden soll, aufweist.

9. Instrument (600) nach einem der Ansprüche 1 bis 4, wobei der Einführervorsatz (604) aufweist:
ein Körperelement (608), in dem ein Längskanal (902) definiert ist;
ein Rohr (610), das an einem nahen Ende des Rohrs (610) mit dem Längskanal (902) des Körperelements (608) gekoppelt ist und mit ihm in Verbindung steht;
ein offenendiges Tablett (1604), das mit einem fernen Ende des Rohrs (610) gekoppelt ist, und
eine Abdeckung (1608), die das offenendige Tablett (1604) umgibt und ein offenes Ende gegenüber dem Rohr (610) hat, wobei das offene Ende mindestens zwei Klappen (1701, 1703) aufweist, von denen eine der mindestens zwei Klappen (1701, 1703) länger ist als die andere der mindestens zwei Klappen (1701, 1703) und im Wesentlichen über ihr liegt, so das ein Raum, der teilweise durch das offenendige Tablett (1604) und die Abdeckung (1608) definiert ist, mindestens teilweise durch die mindestens zwei Klappen (1701, 1703) geschlossen ist.

10. Instrument (600) nach Anspruch 9, wobei der Einführervorsatz ferner aufweist:
ein im Raum angeordnetes Netzhautimplantat (1602).

## Revendications

1. Instrument (600) à utiliser avec un implant médical, comprenant :
un logement (603) comprenant une première extrémité (708), une deuxième extrémité (710) et un canal longitudinal (704) défini dans celle-ci entre la première extrémité (708) et la deuxième extrémité (710), la première extrémité (708) comprenant une première ouverture ;
un élément de nez (730) couplé à la première extrémité du logement (603), comprenant un canal de nez et une surface d'inclinaison (732) ;
un premier élément coulissant (720) disposé de façon mobile dans le canal longitudinal (704) et le canal de nez dans la première ouverture comprenant un corps (722) et au moins deux jambes (724) disposées longitudinalement à une extrémité du corps (722), chaque jambe comprenant une surface d'engagement (726) et se terminant dans une partie pour doigt (728) à une extrémité distale par rapport au corps (722) ; et
une fixation d'applicateur (604) à utiliser en insérant un implant médical dans le corps d'un patient, la fixation d'applicateur (604) étant adaptée pour être couplée à l'élément de nez (730) via la partie pour doigt (728) de chaque jambe (724),
le mouvement du premier élément coulissant (720) le long du canal longitudinal (704) entraînant la mise en prise de la surface de mise en prise (726) de chaque jambe d'au moins deux jambes (724) avec la surface d'inclinaison (732) et ainsi, l'inclinaison de la partie pour doigt (728) de chaque jambe (724) vers l'intérieur, et
la partie pour doigt (728) de chaque jambe (724) étant adaptée pour mettre en prise une surface de la fixation d'applicateur (604) de façon à maintenir la fixation d'applicateur (604) dans une relation fixée par rapport à l'élément de nez (730) lorsque le premier élément coulissant (720) se trouve dans une position initiale.

2. Instrument (600) selon la revendication 1, dans lequel un élément de bouton (606) est présent, raccordé fonctionnellement au premier élément coulissant (720) pour permettre un mouvement actionné par l'utilisateur du premier élément coulissant (720) et un premier élément élastique (740) met en prise l'élément de bouton (606) et le logement (603) de façon à appliquer une force d'inclinaison pour retenir le premier élément coulissant (720) dans sa position initiale.

3. Instrument (600) selon la revendication 1, l'élément de nez (730) comprenant une surface ayant au moins deux cavités (1102) définies dans celle-ci, lesdites au moins deux cavités (1102) étant placées à différentes positions angulaires par rapport à un axe longitudinal (706) du logement (603) de telle sorte qu'un élément en saillie (930) de la fixation d'applicateur peut s'apparier avec l'une desdites au moins deux cavités (1102) pour maintenir ainsi la fixation d'applicateur (604) dans un alignement choisi.

4. Instrument (600) selon l'une quelconque des revendications 1 à 3, comprenant en outre un implant rétinien (1602) disposé dans la fixation d'applicateur (604).

5. Instrument (600) selon l'une quelconque des revendications 1 à 4, dans lequel la fixation d'applicateur (604) comprend :
un corps (608) ayant un canal longitudinal (902) défini dans celui-ci ;
un conduit (610) couplé et en communication avec le canal longitudinal (902) du corps (608) à l'extrémité proximale du conduit (610) ;
un lien (912) disposé dans le conduit (610) et le canal longitudinal se terminant dans un capuchon poussoir (914) à une première extrémité du lien (912) au niveau proximal du corps (608) et se terminant dans un poussoir (1606) à une deuxième extrémité du lien (912) à une extrémité distale du conduit (610) ; et
un élément élastique (928) interposé entre le corps (608) et le couvercle poussoir (914) de telle sorte que le poussoir (1606) soit incliné dans une position rétractée.

6. Instrument (600) selon la revendication 5,
dans lequel un levier (614) et un dispositif de liaison sont aménagés pour conférer un mouvement longitudinal ou axial au deuxième élément coulissant (750) disposé dans le logement (603), et
dans lequel le capuchon poussoir (914), lorsque la fixation d'applicateur (604) est couplée à l'élément de nez (730) met en prise le deuxième élément coulissant (750) de sorte qu'un mouvement longitudinal du deuxième élément coulissant (750) dans le logement soit transmis via le lien de conduit (912) de la fixation d'applicateur (604) pour déplacer le poussoir (1606) à l'extrémité distale du conduit (610).

7. Instrument (600) selon la revendication 5 ou la revendication 6, dans lequel la fixation d'applicateur (604) comprend en outre :
un élément en saillie (930) couplé au corps (608), dans lequel l'élément en saillie (930) est adapté pour s'apparier avec au moins une cavité (1102) disposée dans l'instrument (600) pour maintenir l'alignement de la fixation d'applicateur (604).

8. Instrument (600) selon la revendication 5 ou la revendication 6, dans lequel un plateau à extrémité ouverte (1604) est couplé à l'extrémité distale du conduit (610) de sorte que le poussoir (1606) repose dans le plateau à extrémité ouverte (1604) et dans lequel le plateau à extrémité ouverte (1604) comprend un espace pour tenir l'implant médical à insérer dans le corps du patient.

9. Instrument (600) selon l'une quelconque des revendications 1 à 4, dans lequel la fixation d'applicateur (604) comprend :
un corps (608) ayant un canal longitudinal (902) défini dans celui-ci ;
un conduit (610) couplé et en communication avec le canal longitudinal (902) du corps (608) à l'extrémité proximale du conduit (610) ;
une couverture (1608) enveloppant le plateau à extrémité ouverte (1604) ayant une extrémité ouverte opposée au conduit (610), l'extrémité ouverte comprenant au moins deux panneaux (1701, 1703) dans laquelle l'un desdits au moins deux panneaux (1701, 1703) est plus long que l'autre desdits au moins deux panneaux (1701, 1703) et recouvre substantiellement celle-ci de sorte qu'un espace défini en partie par le plateau à extrémité ouverte (1604) et la couverture (1608) soit au moins partiellement fermé par au moins les deux panneaux (1701, 1703).

10. Instrument (600) selon la revendication 9, dans lequel la fixation d'applicateur comprend en outre :
un implant rétinien (1602) disposé dans l'espace.
